(19) Europäisches Patentamt
European Patent Office
Office européen des brevets

(11) Publication number: **0 204 517**
**A2**

(12) # EUROPEAN PATENT APPLICATION

(21) Application number: 86304089.5

(22) Date of filing: 29.05.86

(51) Int. Cl.⁴: **C 07 D 501/04**
C 07 D 501/26, C 07 D 501/57

(30) Priority: 30.05.85 JP 118625/85

(43) Date of publication of application:
10.12.86 Bulletin 86/50

(84) Designated Contracting States:
AT CH DE FR IT LI NL SE

(71) Applicant: SHIONOGI & CO., LTD.
12, Dosho-machi 3-chome Higashi-ku
Osaka 541(JP)

(72) Inventor: Tsuji, Teruji
1-14-9, Yanagawa-cho
Takatsuki-shi Osaka(JP)

(72) Inventor: Okada, Tetsuo
4-6-6, Akasakadai
Sakai-shi Osaka(JP)

(74) Representative: Bizley, Richard Edward et al,
BOULT, WADE & TENNANT 27 Furnival Street
London EC4A 1PQ(GB)

(54) **Hydroxymethylcephem compounds and their preparation.**

(57) A process for preparing 7β-acylamino-3-hydroxymethyl-3-cephem-4-carboxylic acids, optionally having 7 α-methoxy groups, or their alkali metal salts, comprises treating 7β-acylaminocephalosporanic acids (optionally having 7 α-methoxy groups) or their alkali metal salts with aqueous alkali metal hydroxides in lower alkanols.

Croydon Printing Company Ltd.

0204517

# HYDROXYMETHYLCEPHEM COMPOUNDS AND THEIR PREPARATION

This invention relates to a process for preparing 7β-acylamino-3-hydroxymethyl-3-cephem-4-carboxylic acids optionally having 7α-methoxy groups or their alkali metal salts, which process comprises treating 7β-acylaminocephalosporanic acids optionally having 7α-methoxy groups or their alkali metal salts with an aqueous alkali metal hydroxide in a lower alkanol.

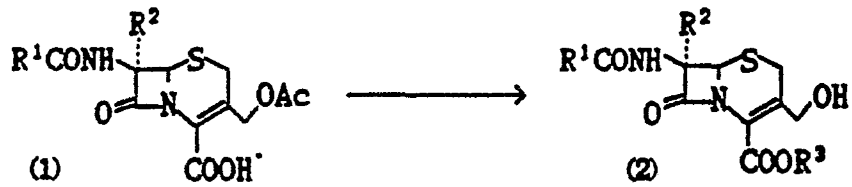

(1) (2)

(wherein $R^1$ is acyl;

$R^2$ is hydrogen or methoxy;

$R^3$ is hydrogen or alkali metal; and

Ac is acetyl)

The objective compounds of this invention (2) were believed to be available by hydrolysis with a base on a large scale only with difficulty due to betalactam fission (Journal of Medicinal Chemistry, Volue 17, page 1312 (1974)) and to practically irreversible lactonization between 3-methylol and 4-carboxy groups with acid

during work up of the reaction mixture. Thus, industrial production has been carried out using enzymes (e.g. Belgian Patent No. 671692 and US Patent No. 3459746). However, such enzymatic processes require tedious handling processes for the voluminous reaction mixture. Recently, alkali metal hydroxides have been used when the 7β-side chain is amino (Japanese Patent Publication (Kokai) 99486/1983; Hungarian Patent Application T 033155). It was said that the success probably resulted from an interaction of 7β-amino with 4-carboxy.

The present invention is based upon the surprising discovery that hydrolysis with aqueous sodium hydroxide in methanol works well even in the case of 7β-acylaminocephalosporanic acids (for which no successful chemical hydrolysis has yet been reported nor industrially operated).

Thus, according to the process of this invention, a 7β-acylaminocephalosporanic acids optionally having a 7α-methoxy group, or an alkali metal salt thereof is treated with an alkali metal hydroxide in a lower alkanol to obtain the corresponding 3-hydroxymethyl-3-cephem-4-carboxylic acid in high yield.

Acyl $R^1CO$ in the above reaction scheme is optionally substituted aliphatic, alicyclic, aromatic, heteroaromatic, or like carboxylic acyl preferably containing 1 to 20 carbon atoms, especially that of the desired objective cephalosporins. It may be acyl as found in the amido side chain of natural or optionally protected synthetic penicillins or cephalosporins.

Typical acyl groups include the following:

1) $R^{10}CH_2CO-$

2) $R^{10}OCH_2CO-$

3) $R^{10}SCH_2CO-$

4) $R^{10}CHCO-$
$\quad\quad R^{11}$

5) $R^{10}CCO-$
$\quad\quad \| $
$\quad\quad R^{12}$

,and 6) $R^{13}CO-$

(wherein $R^{10}$ is hydrogen or an aliphatic, aromatic, heterocyclic, or alicyclic group;

$R^{11}$ is an optionally protected hydroxy, amino, carboxy, sulfo, mercapto, cyano, or like group;

$R^{12}$ is an oxo, thioxo, imino, hydroxyimino, optionally substituted alkoxyimino, alkylidene, or like divalent group;

and $R^{13}$ is hydrogen or an aromatic or heterocyclic group)

The aliphatic group $R^{10}$ may be optionally substituted alkyl, alkenyl, or alkynyl; the aromatic group $R^{10}$ may be optionally substituted phenyl or naphthyl; the heterocyclic group $R^{10}$ may be a five- or six-membered monocyclic or dicyclic heterocyclic group having 1 to 4 nitrogen atoms and/or oxygen or sulfur as the hetero atom; and the alicyclic group $R^{10}$ may be an optionally substituted four- to six-membered optionally 1 or 2 unsaturated cycloalkyl group.

When the group $R^{11}$ is protected, such protection includes that preventing adverse change during synthesis or in the body and for improving physiological or pharmacological characteristics.

Typical examples of the former are optionally substituted alkyl or heterocycle (e.g., t-butyl, tetrahydropyranyl, tetrahydrofuranyl), alkenyl (forming an enol ether or enamine); alkylated or alkoxylated silyl or stannyl; optionally substituted aralkyl (e.g., methoxybenzyl, phenacyl, benzhydryl, trityl); optionally substituted acyl (e.g., alkanoyl, alkenoyl, aroyl, carbonic acyl); and when $R^{11}$ is carboxy, an optionally substituted alkyl, aralkyl, aryl, or the like ester or amide group. The protecting group is a group removable without adverse effects other parts of the molecule.

Typical examples of the latter are (when $R^{11}$ is hydroxy) sulfo, optionally N-substituted carbamoyl, optionally N-substituted sulfamoyl, carbalkoxy, carbaralkoxy, optionally substituted alkanoyl, aralkanoyl, aroyl, or heterocyclic carbonyl; (when $R^{11}$ is amino) alkylsulfonyl, alkyloxoimidazolidinylcarbonyl, diketopiperazinylcarbonyl, alkylureidocarbonyl, or alkylthioureidocarbonyl; and (when $R^{11}$ is carboxy or sulfo) groups removable in the body, i.e., so-called pharmacologically active ester group 1-oxygenated alkyl, aryl, or the like.

The substituent attached to the optionally substituted alkoxyimino or alkylidene $R^{12}$ may be carboxy, esterified or amidated carboxy, hydroxyalkyl, acyloxyalkyl, alkoxy, or the like, and each may be saturated, unsaturated, linear, branched, or cyclic.

The group $R^{13}$ may be hydrogen or an optionally substituted mono- or di-cyclic aryl, heterocyclic group, or a fused ring group

in which the second ring may be non-aromatic.

The groups $R^{10}$ to $R^{13}$ may have a substituent. Typical examples of the substituent include a carbon function (e.g., linear, branched, or cyclic alkyl, acyl, aralkyl, aryl, heteroaromatic, carbamoyl, carboxy, cyano, or the like, each optionally having amino, hydroxy, oxo, carboxy, or the like); a nitrogen function (e.g., amino, acylamino, guanidyl, ureido, alkylamino, aralkylamino, isothiocyano, isocyano, nitro, nitroso); an oxygen function (e.g., hydroxy, alkoxy, aralkoxy, aryloxy, heterocyclic oxy, oxo, cyanato, acyloxy) or a corresponding sulfur function; sulfo; sulfamoyl; halogen; and substituted silyl or stannyl.

The aryl part in the groups $R^{10}$ to $R^{13}$ may be carbocyclic or heterocyclic, monocyclic or dicyclic, and five- or six-membered aryl optionally substituted as given above. Typical heterocyclic groups incude furyl, thienyl, pyrrolyl, oxazolyl, thiazolyl, imidazolyl, oxadiazolyl, thiadiazolyl, triazolyl, thiatriazolyl, tetrazolyl, pyridyl, quinolyl, pyridopyridyl, and the like.

The alkyl part in the groups $R^{10}$ to $R^{13}$ may be saturated, unsaturated, linear, branched, or cyclic.

The lower alkanol used may preferably be methanol, ethanol, propanol, butanol, isobutanol, t-butanol, or like 1C to 5C alkanol, especially methanol or ethanol.

The ratio of the lower alkanol to water in the aqueous alkali metal hydroxide is preferably 1:4 to 4:1, especially 1:3 to 3:1.

When the ratio of both solvents is beyond the above range, then the the reaction is slower and the formation of by-products is significant.

The alkali metal hydroxide may be lithium hydroxide, sodium hydroxide, or potassium hydroxide. At least 1 molar equivalent of the alkali metal hydroxide is used for the hydrolysis and to neutralize the resulting acetic acid. If the starting cephalosporanic acid is free, then another 1 molar equivalent of the alkali metal hydroxide is necessary for neutralizing the carboxy group.

Under typical conditions for this invention, aqueous alkal metal hydroxide (1 to 8 molar equivalents) is added to a solution of 7β-acylaminocephalosporanic acid optionally having a 7α-methoxy group, in a lower alkanol, especially methanol or ethanol, (3 to 10 parts by weight of the starting cephem compound and in 1 : 3 to 3 : 1 part by weight of water in the aqueous alkali metal hydroxide), and the mixture is stirred for 5 minutes to 3 hours at -70 °C to 0°C, preferably at -40°C to -10°C to give alkali metal·7β-acylamino-3-hydroxymethyl-3-cephem-4-carboxylate (optionally having 7α-methoxy) in up to 99 % yield. The product may be recovered from the reaction mixture by desalting and/or lyophilizing.

When the said reaction mixture is neutralized slowly (preferably with mineral acid) with stirring at a low temperature (especially at -10°C to 5°C) to prevent the lactonization, the corresponding free carboxylic acid is obtained in high yield.

To the reaction medium may be added an inert polar solvent as

a co-solvent. Typical co-solvents include ethers (e.g. dioxane, tetrahydrofuran), ketones (e.g., acetone, cyclohexanone), nitriles (e.g., acetonitrile), amides(e.g., formamide, dimethylacetamide, hexamethylphosphorotriamide), sulfoxides(e.g., dimethylsulfoxide), organic bases (e.g., diethylamine, triethylamine), alcohols (e.g., methanol, ethanol, propanol, hexanol, octanol, benzyl alcohol), and like industrial solvents, or a mixture of two or more of these.

The objective product may be recovered from the reaction mixture after removing contaminants (e.g., unreacted starting material, by-products, solvents) by a conventional method (e.g., extraction, washing, concentration, precipitation, filtration, drying) and purified by usual work up procedures (e.g., adsorption elution, precipitation, separation, crystallization, lyophilization).

Some of the objective compounds are novel. The following are representative examples:

7β-difluoromethylthio-acetylamino-7α-methoxy-3-hydroxymethyl-3-cephem-4-carboxylic acid,

7β-[2-(2-tert-butoxycarbonylamino-4-thiazolyl)-2-(methoxyimino)acetylamino]-3-hydroxymethyl-3-cephem-4-carboxylic acid,

7β-[2-(2-t-butoxycarbonylamino-4-thiazolyl)-2-butenoylamino]-3-hydroxymethyl-3-cephem-4-carboxylic acid,

7β-[2-(2-benzyloxycarbonylamino-4-thiazolyl)-4-tert-butoxycarbonyl-2-butenoylamino]-3-hydroxymethyl-3-cephem-4-carboxylic acid,

7β-[2-(2-tert-butoxycarbonylamino-4-thiazolyl)-2-pentenoyl-amino]-3-hydroxymethyl-3-cephem-4-carboxylic acid sodium salt,

7β-[2-tert-butoxycarbonylamino-4-thiazolyl)-2-hexenoylamino]-3-hydroxymethyl-3-cephem-4-carboxylic acid,

7β-[2-(2-t-butoxycarbonylamino-4-thiazolyl)-4-methyl-2-pentenoylamino]-3-hydroxymethyl-3-cephem-4-carboxylic acid, and

7β-[2-(2-t-butoxycarbonylamino-4-thiazol-yl)-4-cyclopentyl-2-butenoylamino]-3-hydroxymethyl-3-cephem-4-carboxylic acid, and cis- and trans-isomers and alkali metal salts of these.

The merit of this invention is that the betalactam ring is intact and lactonization is hardly observable, even after work up of the reaction mixture.

As explained above, the known hydrolysis with a base produces 7β-amino-3-hydroxymethyl-3-cephem-4-carboxylic acid. When this is used as the starting material for producing cephalosporins, it has to be acylated at its 7β-amino position. In order to avoid side reactions, the acylation has to be done under Schotten-Baumann conditions. Thus, when the acyl to be introduced cannot form an acid chloride efficiently, the known hydrolysis is not useful. In the method of this invention, the acyl is already present in the starting material, and so the type of acyl group can be chosen more freely than in the prior art. This is the second merit

of this invention.

The objective 7$\beta$-acylamino-3-hydroxymethyl-3-cephem-4-carboxylic acids (optionally having 7$\alpha$-methoxy) and their alkali metal saltsare useful as starting materials for producing, for example, cephalosporins having a 3-carbamoyloxymethyl group (e.g. cefoxitin, cefuroxime), by a well know acylation procedure.

The following Examples illustrate the invention. The physical constants of the products are listed on Table 1. In the table, IR shows wave number $\nu$ in $cm^{-1}$ and NMR shows chemical shift $\delta$ in ppm and coupling constant J in Hz.

In the Examples, the amounts shown by part are the weight of the substance per 1 part by weight of the starting betalactam. The amount shown by equivalent is the molar ratio of the substance per 1 mole of the starting betalactam.

The reaction employed in the following Examples is represented by the following equation:

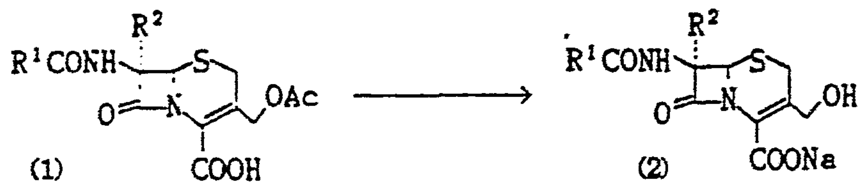

Example 1 ($R^1CO$ = formyl, $R^2$ = hydrogen)

To a solution of the starting cephalosporanic acid (1) in methanol (4.3 parts) is added aqueous 1N-sodium hydroxide (1 equivalent) at -10°C, and the mixture is stirred for 10 minutes. Then, another 1 equivalent of aqueous 1N-sodium hydroxide is added and the mixture is stirred for 20 minutes. The reaction mixture is diluted with water (17 parts) and concentrated under reduced pressure. The remaining aqueous solution is passed through a column of stylene-divinylbenzene copolymer (75 parts) (Synthetic adsorbent Diaion HP-20 distributed by Mitsubishi Kasei Kogyo K.K.) to trap the product. The product is eluted with water. The eluate is lyophilized to give 7$\beta$-formamido-3-hydroxymethyl-3-cephem-4-carboxylic acid sodium salt (2) (0.61 parts). Yield: 65%.

Example 2 ($R^1CO$ = difluoromethylthioacetyl, $R^2$ = methoxy)

To a solution of the starting cephalosporanic acid (1) in methanol (20 parts) is added aqueous 1N-sodium hydroxide (2.5 equivalents) at -20°C, and the mixture is stirred for 40 minutes at -20 to -15°C. The reaction mixture is neutralized with 1N-hydrochloric acid (1.5 equivalents) and concentrated under reduced pressure to remove methanol. The remaining aqueous solution is passed through a column of stylene-divinylbenzene copolymer (88 parts) (Synthetic adsorbent Diaion HP-20 distributed by Mitsubishi Kasei Kogyo K.K.) to trap the product. The product is eluted with water. The eluate is lyophilized to give 7β-difluoromethylthioacetylamino-7α-methoxy-3-hydroxymethyl-3-cephem-4-carboxylic acid sodium salt (2) as powder (0.83 parts). Yield: 87 %.

Another reaction mixture is neutralized with 1N-hydrochloric acid cautiously to adjust pH to 2 to 3 and extracted several times with ethyl acetate to give the crude carboxylic acid in 60 % yield.

Example 3 . ($R^1CO$ = 2-thienylacety, $R^2$ = hydrogen)

To a suspension of the starting cephalosporanic acid (1) sodium salt in a mixture of water (6 parts) and methanol (8.2 parts) is added aqueous 1N-sodium hydroxide (0.5 equivalents) at -10°C, and the mixture is stirred for 30 minutes. Then, another 0.5 equivalents of aqueous 1N-sodium hydroxide is added and the mixture is stirred for 30 minutes. The reaction mixture is mixed with 1N-hydrochloric acid (2 parts), aqueous saturated sodium hydrogen

carbonate (10 parts) and water (20 parts) and concentrated under reduced pressure. The remaining aqueous solution is passed through a column of stylene-divinylbenzene copolymer (32 parts) (Synthetic adsorbent Diaion HP-20 distributed by Mitsubishi Kasei Kogyo K.K.) to trap the product. The product is eluted with a mixture of methanol and water (1 : 2). The eluate is lyophilized to give 7$\beta$-(2-thienyl)acetylamino-3-hydroxymethyl-3-cephem-4-carboxylic acid sodium salt (2) (0.74 parts). Yield: 83 %.

Example 4 ($R^1CO$ = 2-(2-furyl)-2-methoxyiminoacetyl, $R^2$ = H)

To a solution of the starting cephalosporanic acid (1) in methanol (10 parts) is added aqueous 1N-sodium hydroxide (5.7 parts) (2.4 equivalent) at -15°C, and the mixture is stirred for 20 minutes. The reaction mixture is neutralized with 1N-hydrochloric acid to pH 7, diluted with water (20 parts), and concentrated under reduced pressure. The remaining aqueous solution is passed through a column of stylene-divinylbenzene copolymer (50 parts) (Synthetic adsorbent Diaion HP-20 distributed by Mitsubishi Kasei Kogyo K.K.) to trap the product. The product is eluted with water to a mixture of methanol and water (1 : 5). The eluate is lyophilized to give 7$\beta$-[2-(2-furyl)-2-(methoxyimino)acetylamino]-3-hydroxymethyl-3-cephem-4-carboxylic acid sodium salt (2) (0.74 parts). Yield: 78 %.

Example 5 ($R^1CO$ = 2-(2-t-butoxycarbonylamino-4-thiazolyl)-

2-(methoxyimino)acetyl, $R^2$ = hydrogen)

To a solution of the starting cephalosporanic acid (1) in methanol (6.9 parts) is added aqueous 1N-sodium hydroxide (1 equivalent) at -10°C, and the mixture is stirred for 1 hour. Then, another aqueous 1N-sodium hydroxide (1 equivalent) is added and the mixture is stirred for 10 minutes. To the reaction mixture are added 1N-hydrochloric acid (2 parts), aqueous saturated sodium hydrogen carbonate (10 parts), and water (30 parts), and the mixture is concentrated under reduced pressure. The remaining aqueous solution is passed through a column of stylene-divinylbenzene copolymer (45 parts) (Synthetic adsorbent Diaion HP-20 distributed by Mitsubishi Kasei Kogyo K.K.) to trap the product. The product is eluted with a mixture of methanol and water (1 : 2 to 1 : 1). The eluate is lyophilized to give 7β-[2-(2-tert-butoxycarbonylamino-4-thiazolyl)-2-(methoxyimino)acetylamino]-3-hydroxymethyl-3-cephem-4-carboxylic acid sodium salt (2) (0.67 parts). Yield: 70 %.

Example 6 ( $R^1CO$ = cis-2-(2-t-butoxycarbonylamino-4-thiazolyl)-2-butenoyl, $R^2$=hydrogen)

To a solution of the starting cephalosporanic acid (1) in methanol (7.5 parts) is added aqueous 1N-sodium hydroxide (4.7 parts, 2.5 equivalents) at -25°C, and the mixture is stirred for 70 minutes. The reaction mixture is neutralized with 1N-hydrochloric acid to pH 6.5, diluted with ethyl acetate (7.5 parts) and

water (1.9 parts), and concentrated under reduced pressure. The resulting aqueous solution is acidified with 1Nhydrochloric acid to pH 2.5 and extracted with ethyl acetate. The extract is washed with water, dried, and concentrated under reduced pressure to give 7β-[cis-2-(2-t-butoxycarbonylamino-4-thiazolyl)-2-butenoylamino]-3-hydroxymethyl-3-cephem-4-carboxylic acid (2) (0.90 parts). Yield: 97 %.

Example 7 ($R^1CO$ = trans-2-(2-t-butoxycarbonylamino-4-thiazolyl)-2-butenoyl, $R^2$ = hydrogen)

To a solution of the starting cephalosporanic acid (1) in methanol (6.9 parts) is added aqueous 1N-sodium hydroxide (1 equivalent) at -15°C, and the mixture is stirred for 20 minutes. Then, another aqueous 1N-sodium hydroxide (1 equivalent) is added and the mixture is stirred for 10 minutes. Then, further aqueous 1N-sodium hydroxide (0.5 equivalent) is added and the mixture is stirred for 30 minutes. To the reaction mixture are added 1N-hydrochloric acid (3 parts), aqueous saturated sodium hydrogen carbonate (10 parts), and water (30 parts), and the mixture is concentrated under reduced pressure. The remaining aqueous solution is passed through a column of stylene-divinylbenzene copolymer (45 parts) (Synthetic adsorbent Diaion HP-20 distributed by Mitsubishi Kasei Kogyo K.K.) to trap the product. The product is eluted with a mixture of methanol and water (1 : 2 to 1 : 1). The eluate is lyophilized to give 7β-[trans-2-(2-tert-butoxycarbonylamino-4-thiazolyl)-2-butenoylamino]-3-hydroxymethyl-3-cephem-4-carboxylic acid sodium salt (2) (0.69 parts). Yield: 72 %.

Example 8 ($R^1CO$ = cis and trans-2-(2-benzyloxycarbonylamino-4-thiazolyl)-4-t-butoxycarbonyl-2-butenoyl, $R^2$ = hydrogen)

To a solution of the starting cephalosporanic acid (1) in methanol (10 parts) is added aqueous 1N-sodium hydroxide (2.5 equivalents) at -40°C, and the mixture is stirred for 40 minutes at -25°C. The reaction mixture is neutralized with 4N-hydrochloric acid to pH 6 to 7, concentrated to a half volume, washed with ether, and passed through a column of stylene-divinylbenzene copolymer (10 parts) (Synthetic adsorbent Diaion HP-20 distributed by Mitsubishi Kasei Kogyo K.K.) to trap the product. The product is eluted with a mixture of methanol and water (3:10 to 4:1). The eluate is concentrated and lyophilized to give a mixture of cis- and trans-7β-[2-(2-benzyloxycarbonylamino-4-thiazolyl)-4-tert-butoxycarbonyl-2-butenoylamino]-3-hydroxymethyl-3-cephem-4-carboxylic acid sodium salts (2) (0.45 parts). Yield: 49 %.

Example 9 ($R^1CO$ = cis-2-(2-t-butoxycarbonylamino-4-thiazolyl)-2-pentenoyl, $R^2$ = hydrogen)

To a solution of the starting cephalosporanic acid (1) in methanol (6 parts) is added aqueous 1N-sodium hydroxide (1.4 parts) (1.0 equivalent) at -15°C, and the mixture is stirred for 5 minutes. Then, another aqueous 1N-sodium hydroxide (2.2 parts)

(1.5 equivalent) is added and the mixture is stirred for 20 minutes. The reaction mixture is neutralized with 1N-hydrochloric acid to pH 6, diluted with water (20 parts) and concentrated under reduced pressure. The remaining aqueous solution is passed through a column of stylene-divinylbenzene copolymer (40 parts) (Synthetic adsorbent Diaion HP-20 distributed by Mitsubishi Kasei Kogyo K.K.) to trap the product. The product is eluted with a mixture of methanol and water (1 : 3 to 1 : 2). The eluate is lyophilized to give 7β-[cis-2-(2-tert-butoxycarbonylamino-4-thiazolyl)-2-penten-oylamino]-3-hydroxymethyl-3-cephem-4-carboxylic acid sodium salt (2) (0.65 parts). Yield: 67 %.

Example 10 ( $R^1$CO= cis-2-(2-t-butoxycarbonylamino-4-thiazolyl)-2-hexenoyl, $R^2$=hydrogen)

To a solution of the starting cephalosporanic acid (1) in methanol (9.8 parts) is added aqueous 1N-sodium hydroxide (4.5 parts) (2.5 equivalent) at -35°C, and the mixture is stirred for 100 minutes. The reaction mixture is neutralized with 1N-hydrochloric acid to pH 7, and separating precipitate is collected by filtration. This is dissolved in aqueous sodium hydrogen carbonate (1 equivalent) and lyophilized to give 7β-[2-tert-butoxycarbonylamino-4-thiazolyl)-2-hexenoylamino]-3-hydroxymethyl-3-cephem-4-carboxylic acid sodium salt (2) (0.69 parts). Yield: 70 %.

Example 11 ( $R^1CO$= cis-2-(2-t-butoxycarbonylamino-4-thiazolyl)-4-methyl-2-pentenoyl, $R^2$=hydrogen)

To a solution of the starting cephalosporanic acid (1) in methanol (9.4 parts) is added aqueous 1N-sodium hydroxide (4.5 parts) (2.5 equivalent) at -35°C, and the mixture is stirred for 110 minutes. The reaction mixture is neutralized with 1N-hydrochloric acid to pH 7, made alkaline with aqueous sodium hydrogen carbonate to pH 9, and concentrated under reduced pressure. The resulting aqueous solution is remove methanol. The remaining aqueous solution is passed through a column of stylene-divinylbenzene copolymer (Synthetic adsorbent Diaion HP-20 distributed by Mitsubishi Kasei Kogyo K.K.) to trap the product. The product is eluted with a mixture of methanol and water (1:5). The eluate is lyophilized to give 7β-[cis-2-(2-t-butoxycarbonylamino-4-thiazolyl)-4-methyl-2-pentenoylamino]-3-hydroxymethyl-3-cephem-4-carboxylic acid sodium salt (2) (0.61 parts). Yield: 63 %.

Example 12 ( $R^1CO$= cis-2-(2-t-butoxycarbonylamino-4-thiazolyl)-4-cyclopentyl-2-butenoyl, $R^2$=hydrogen)

To a solution of the starting cephalosporanic acid (1) in methanol (8.3 parts) is added aqueous 1N-sodium hydroxide (4.2 parts, 2.5 equivalents) at -20°C to -25°C, and the mixture is stirred for 60 minutes. The reaction mixture is neutralized with 1N-hydrochloric acid to pH 7 and concentrated under reduced pressure. The residual aqueous solution is acidified to pH 2.6

and extracted with ethyl acetate. The extract is washed with water and concentrated under reduced pressure. The resulting material is dissolved in aqueous sodium hydrogen carbonate (10 parts, 1 molar equivalent) and lyophilized to give 7β-[cis-2-(2-t-butoxycarbonylamino-4-thiazolyl)-4-cyclopentyl-2-butenoylamino]-3-hydroxymethyl-3-cephem-4-carboxylic acid sodium salt (2) (0.90 parts). Yield: 91 %.

Table 1 ( Part 1 )

Physical constants of 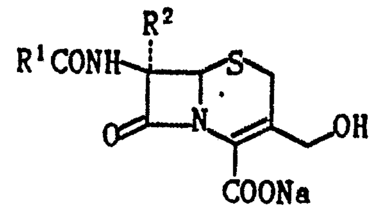

| No. | $R^1CO$ | $R^2$ | IR(KBr): $cm^{-1}$ | NMR($D_2O$): δ ( TMS external standard ) ppm |
|---|---|---|---|---|
| 1 | formyl | H | 3400, 1760, 1672, 1600. (1 hydrate) | 3.92, 4.12(ABq, J=19Hz, 2H), 4.73(s, 2H), 5.60(d, J=5Hz, 1H), 6.19(d, J=5Hz, 1H), 8.70 (s, 1H). |
| 2 | difluoromethylthio-acctyl [free acid] | $CH_3O$ | 3400~3280, 1760, 1677, 1600, 1530, 1070, 1025. | 3.86, 4.11(ABq, J=18.9Hz, 2H), 4.04(s, 3H), 4.73(s, 2H), 5.65(s, 1H), 7.61(t, J=55.5Hz, 1H). [Na salt] |
| 3 | 2-thienylacetyl | H | 3400, 3280, 1750, 1657, 1600, 1550. (1 hydrate) | 3.84, 4.00(ABq, J=18Hz, 2H), 4.30(s, 2H), 4.68(s, 2H), 5.48(d, J=4Hz, 1H), 6.03(d, J=4Hz, 1H), 7.45(m, 2H), 7.74(m, 1H). |

Table 1 (Part 2)

| No. | $R^1CO$ | $R^2$ | IR(KBr): $cm^{-1}$ | NMR($D_2O$): $\delta$ (TMS external standard) ppm |
|---|---|---|---|---|
| 4 | 2-(2-furyl)-2-(methoxyimino)acetyl | H | 3400, 1760, 1665, 1600, 1535. | 3.89, 4.07(ABq, J=19Hz, 2H), 4.40(s, 3H), 4.65, 4.73(ABq, J=14Hz, 2H), 5.60(d, J=5 Hz, 1H), 6.20(d, J=5Hz, 1H), 7.02(dd, J=2Hz, J=4Hz, 1H), 7.28(d, J=4Hz, 1H), 8.11 (d, J=2Hz, 1H). |
| 5 | 2-(2-t-butoxycarbonyl-aminothiazol-4-yl)-2-(methoxyimino)acetyl | H | 3400, 1762, 1721, 1670, 1600, 1551. (anhydrous) | 3.87, 4.08(ABq, J=21Hz, 2H), 4.41(s, 3H), 4.69(s, 2H), 5.58(d, J=4Hz, 1H), 6.19(d, J=4Hz, 1H), 7.78(s,1H). |
| 6 | cis-2-(2-t-butoxy-carbonylaminothiazol-4-yl)-2-butenoyl [free acid] | H | nd. | 1.54(s, 9H), 2.02(d, J=8.0Hz, 3H), 3.59(s, 2H), 4.15, 4.51(ABq, J=13.5Hz, 2H), 5.04 (d, J=5.0Hz, 1H), 5.85(d, J=5.0Hz, 1H), 6.52(q, J=8.0Hz, 1H), 6.77(s, 1H). |
| 7 | trans-2-(2-t-butoxy-carbonylaminothiazol-4-yl)-2-butenoyl | H | 3400, 1755, 1722, 1665, 1605, 1550. (0.5 hydrate) | 1.93(s, 9H), 2.29(d, J=7Hz, 3H), 3.81, 4.06(ABq, J= 19Hz, 2H), 4.70(s, 2H), 5.53 (d, J=4Hz, 1H), 6.11(d, J=4Hz, 1H), 7.4(m, 1H), 7.43(s, 1H). |

Table 1 ( Part 3 )

| No. | $R^1CO$ | $R^2$ | IR(KBr): $cm^{-1}$ | NMR($D_2O$): δ ( TMS external standard ) ppm |
|---|---|---|---|---|
| 8 | cis-2-(2-benzyloxy-carbonylaminothiazol-4-yl)-4-tert-butoxy-carbonyl-2-butenoyl | H | nd. | 1.45(s, 9H), 3.3~4.5(m, 6H), 4.95~5.10(m, 1H), 5.23(s, 2H), 5.65~5.80(m,1H), 6.6~7.0 (m, 1H), 7.09(s, 1H), 7.2 ~ 7.5(m, 5H). [$CD_3OD$] |
| 9 | cis-2-(2-t-butoxy-carbonylaminothiazol-4-yl)-2-pentenoyl | H | 3400, 1756, 1721, 1656, 1600, 1548. | 1.45(t, J=7Hz, 3H), 1.88(s, 9H), 2.66(m, 2H), 3.84, 4.04(ABq, J=18Hz,2H), 4.67(s, 2H), 5.56(d, J=5Hz, 1H), 6.18(d, J=5Hz, 1H), 6.82(t, J=8Hz, 1H), 7.24(s, 1H). |
| 10 | cis-2-(2-t-butoxy-carbonylaminothiazol-4-yl)-2-hexenoyl | H | 3220, 1755, 1725, 1660, 1605, 1550. | 0.95(t, J=8Hz, 3H), 1.55(s, 9H), 2.35(brq, J=8Hz, 2H), 3.52(brs, 2H), 3.82(brs, 2H), 5.03(d.J=5Hz, 1H), 5.79(d, J=5Hz, 1H), 6.44(t, J=8Hz, 1H), 6.79(s, 1H). |
| 11 | cis-2-(2-t-butoxy-carbonylaminothiazol-4-yl)-4-methyl-2-pentenoyl | H | 3250, 1755, 1725, 1660, 1600. | 1.06(d, J=7Hz, 6H), 1.53(s, 9H), 2.5~3.1 (m, 1H), ~3.45(m, 2H), 3.81(s, 2H), 5.02 (d, J=5Hz, 1H), 5.77(d, J=5Hz, 1H), 6.22 (d, J= 10Hz, 1H), 6.80(s, 1H). |

Table 1 ( Part 4 )

| No. | $R^1CO$ | $R^2$ | IR(KBr): $cm^{-1}$ | NMR($D_2O$): δ ( TMS external standard ) ppm |
|---|---|---|---|---|
| 12 | cis-2-(2-t-butoxy-carbonylaminothiazol-4-yl)-4-cyclopentyl-2-butenoyl | H | nd. | TLC Rf 0.45 ($SiO_2$/EtOAc-HOAc-$H_2O$ = 8:1:1). |

CLAIMS:

0204517

1. A process for preparing a 7β-acylamino-3-hydroxymethyl-3-cephem-4-carboxylic acid, optionally having a 7α-methoxy group, or its alkali metal salt comprising treating a 7β-acylaminocephalosporanic acid, optionally having a 7α-methoxy group, with aqueous alkali metal hydroxide in a lower alkanol.

2. A process as claimed in claim 1, wherein the acyl group has from 1 to 20 carbon atoms.

3. A process as claimed in claim 1 or claim 2, wherein the alkali metal hydroxide is sodium hydroxide.

4. A process as claimed in any one of claims 1 to 3, wherein from 1 to 8 molar equivalents of the alkali metal hydroxide are used.

5. A process as claimed in any one of claims 1 to 4, wherein the lower alkanol is a 1C to 5C alkanol, preferably methanol.

6. A process as claimed in any one of claims 1 to 5, wherein the ratio of water and alkanol, preferably methanol, is between 1:3 and 3:1.

7. A process as claimed in any one of claims 1 to 6, wherein the ratio of lower alkanol to the starting cephem is from 1:3 to 1:10 weight by weight.

8. A process as claimed in any one of claims 1 to 7, wherein the treatment is carried out at a temperature between -40°C and 0°C for from 5 minutes to 3 hours.

9. A process as claimed in any one of claims 1 to 8, wherein the product is neutralized to give the corresponding free acid.

10. A compound selected from the group consisting of:

7$\beta$-difluoromethylthio-acetylamino-7$\alpha$-methoxy-3-hydroxymethyl-3-cephem-4-carboxylic acid;

7$\beta$-[2-(2-tert-butoxycarbonylamino-4-thiazolyl)-2-(methoxyimino)acetylamino]-3-hydroxymethyl-3-cephem-4-carboxylic acid;

7$\beta$-[2-(2-t-butoxycarbonylamino-4-thiazolyl)-2-butenoylamino]-3-hydroxymethyl-3-cephem-4-carboxylic acid;

7$\beta$-[2-(2-benzyloxycarbonylamino-4-thiazolyl)-4-tert-butoxycarbonyl-2-butenoylamino]-3-hydroxymethyl-3-cephem-4-carboxylic acid;

7$\beta$-[2-(2-tert-butoxycarbonylamino-4-thiazolyl)-2-pentenoylamino]-3-hydroxymethyl-3-cephem-4-carboxylic acid sodium salt;

7$\beta$-[2-tert-butoxycarbonylamino-4-thiazolyl)-2-hexenoylamino]-3-hydroxymethyl-3-cephem-4-carboxylic acid;

7$\beta$-[2-(2-t-butoxycarbonylamino-4-thiazolyl)-4-methyl-2-pentenoylamino]-3-hydroxymethyl-3-cephem-4-carboxylic acid; and

7$\beta$-[2-(2-t-butoxycarbonylamino-4-thiazol-yl)-4-cyclopentyl-2-butenoylamino]-3-hydroxymethyl-3-cephem-4-carboxylic acid;

and cis- and trans-isomers and alkali metal salts thereof.

11. The use of a 7β-acylaminocephalosporanic acid, optionally having a 7α-methoxy group, or an alkali metal salt thereof which has been prepared by a process as claimed in any one of claims 1 to 9 or which is claimed in claim 10 in producing a cephalosporin, e.g. a cephalosporin having a 3-carbamoyloxmethyl group, or in the production of a therapeutic formulation for the treatment or prophylaxis of bacterial infections, said formulation optionally containing a diluent, carrier or excipient and/or being in unit dosage form.